# DEMANDE DE BREVET EUROPEEN

(11) **EP 1 593 367 A1**
(43) Date de publication de la demande: **09.11.2005**
(21) Numéro de dépôt: 05290964.5
(22) Date de dépôt: 03.05.2005
(51) Int. Cl.: A61K 7/11

(54) **Composition conditionnée dans un dispositif aérosol, comprenant un polymère fixant anionique, une silicone oxyalkylénée en position Alpha et Omega de la chaîne siliconée, et un agent propulseur**

(30) Priorité: 07.05.2004 FR 0404991
(71) Demandeur: L'OREAL, 75008 Paris (FR)
(72) Inventeur: Gringore, Charles, 75017 Paris (FR); Pataut, Francoise, 75017 Paris (FR)
(74) Mandataire: Casalonga, Axel

(57) **Abrégé**

La présente invention concerne une composition conditionnée dans un dispositif aérosol, comprenant dans un milieu cosmétiquement acceptable, au moins un polymère fixant anionique, au moins une silicone oxyalkylénée en positions α et ω de la chaîne siliconée, et au moins un agent propulseur. Elle concerne également son utilisation pour le coiffage des cheveux.

## Description

La présente invention est relative à une composition conditionnée dans un dispositif aérosol comprenant un polymère fixant anionique, une silicone oxyalkylénée en α et ω, un agent propulseur, à un procédé de traitement cosmétique des cheveux et à une utilisation en tant que produit de coiffage.

Dans le domaine du coiffage, l'utilisation de sprays aérosols est bien connue. Il s'agit de formulations conditionnées dans des dispositifs aérosols qui, à la sortie du dispositif aérosol, sont sous forme de fines gouttelettes après pulvérisation et forment ainsi un "spray".

Ces formulations comprenant généralement un polymère fixant, sont utilisées pour structurer la coiffure et lui apporter une tenue durable.

Cependant, certains polymères fixants entraînent un durcisssement de la chevelure. Cet inconvénient conduit à une coiffure figée et à un démêlage souvent difficile en fin de journée, les cheveux présentant un toucher sec.

La Demanderesse a trouvé de manière surprenante et inattendue que l'association particulière d'un polymère fixant anionique, d'une silicone particulière telle que décrite ci-dessous et d'un agent propulseur permettait d'obtenir une chevelure ne présentant pas les inconvénients ci-dessus.

En effet, cette association permet d'obtenir une surface de la chevelure plus lisse et les cheveux sont maintenus dans la forme désirée sans être figés ni durcis.

En outre, le démêlage en fin de journée est facilité et les cheveux sont souples et lisses.

L'invention a donc pour objet une composition conditionnée dans un dispositif aérosol comprenant dans un milieu cosmétiquement acceptable, au moins un polymère fixant anionique, au moins une silicone oxyalkylénée en positions α et ω de la chaîne siliconée, et au moins un agent propulseur.

Un autre objet de la présente invention consiste en un procédé de traitement cosmétique des cheveux mettant en oeuvre la composition selon l'invention.

L'invention a encore pour objet une utilisation de la composition selon l'invention comme produit de coiffage.

D'autres objets, caractéristiques, aspects et avantages de l'invention apparaîtront encore plus clairement à la lecture de la description et de l'exemple qui suit.

Selon l'invention, la composition conditionnée dans un dispositif aérosol comprend dans un milieu cosmétiquement acceptable, au moins un polymère fixant anionique, au moins une silicone oxyalkylénée en positions α et ω de la chaîne siliconée, et au moins un agent propulseur.

Par milieu cosmétiquement acceptable, on entend au sens de la présente invention un milieu compatible avec les cheveux.

Par polymère fixant, on entend au sens de la présente invention tout polymère susceptible d'apporter une forme à une chevelure ou de maintenir une forme donnée.

Les polymères fixants anioniques utilisables dans la présente invention peuvent être des polymères comportant des groupements dérivés d'acide carboxylique, sulfonique ou phosphorique. Ces derniers ont une masse moléculaire moyenne en nombre comprise entre environ 500 et 50 000 000.

Les groupements carboxyliques sont apportés par des monomères mono- ou diacides carboxyliques insaturés tels que ceux répondant à la formule : 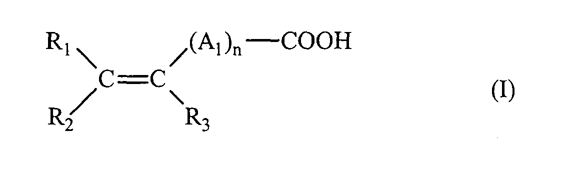 dans laquelle n est un nombre entier de 0 à 10, A₁ désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, R₁ désigne un atome d'hydrogène, un groupement phényle ou benzyle, R₂ désigne un atome d'hydrogène, un groupement alkyle inférieur en C₁₋₄, par exemple un groupement méthyle ou éthyle, ou carboxyle, R₃ désigne un atome d'hydrogène, un groupement alkyle inférieur, un groupement -CH₂-COOH, phényle ou benzyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL® E ou K par la société ALLIED COLLOID, et ULTRAHOLD® par la société BASF, les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois n^{os} 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀, par exemple, de lauryle, tels que celui commercialisé par la société ISP sous la dénomination ACRYLIDONE® LM et les terpolymères acide méthacrylique/acrylate d'éthyle/acrylate de tertiobutyle tels que le produit commercialisé sous la dénomination LUVIMER® 100 P par la société BASF.
   On peut aussi citer les copolymères acide méthacrylique/acide acrylique/acrylate d'éthyle/méthacrylate de méthyle en dispersion aqueuse, commercialisé sous la dénomination AMERHOLD® DR 25 par la société AMERCHOL.
C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les brevets français n^{os} 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch.
D) Les copolymères d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n^{os} 2 047 398, 2 723 248, 2 102 113, le brevet GB n° 839 805. Des produits commerciaux sont notamment ceux vendus sous les dénominations GANTREZ® AN ou ES par la société ISP.
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,
   les fonctions anhydrides de ces copolymères étant éventuellement monoestérifiées ou monoamidifiées.
   Ces polymères sont par exemple décrits dans les brevets français n^{os} 2 350 384 et 2 357 241 de la demanderesse.
E) Les polyacrylamides comportant des groupements carboxylates.
F) Les homopolymères et copolymères comprenant des groupements sulfoniques ; ce sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
- les sels de l'acide polystyrène-sulfonique tels que les sels de sodium vendus par exemple sous les dénominations Flexan® 500 et Flexan® 130 par National Starch. Ces composés sont décrits dans le brevet FR 2 198 719.
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER HSP 1180 par Henkel.

On peut aussi utiliser des polyuréthanes anioniques fixants comme polymères fixants anioniques.

Les polyuréthanes anioniques peuvent être formés par un arrangement de blocs, cet arrangement étant obtenu notamment à partir de :
(1) au moins un composé qui contient deux ou plus de deux atomes d'hydrogène actifs par molécule ;
(2) au moins un diol ou un mélange de diols contenant des fonctions acides ou leurs sels ; et
(3) au moins un di- ou polyisocyanate.

Avantageusement, les composés (1) sont choisis dans le groupe comprenant les diols, les diamines, les polyesterols, les polyétherols ou leurs mélanges.

Les composés (1) qui sont préférés sont les polyéthylène-glycols et les polypropylèneglycols linéaires, en particulier ceux qui sont obtenus par réaction de l'oxyde d'éthylène ou de propylène avec l'eau ou du diéthylène ou du dipropylèneglycol en présence d'hydroxyde de sodium en tant que catalyseur. Ces polyalkylène-glycols ont généralement une masse moléculaire comprise entre environ 600 et 20 000.

D'autres composés organiques préférés sont ceux qui ont des groupes mercapto, amino, carboxyle ou hydroxyle. Parmi ceux-ci, on cite plus particulièrement les composés polyhydroxylés tels que les polyéther-diols, les polyester-diols, les polyacétal-diols, les polyamide-diols, les polyester-polyamide-diols, les poly(alkylène éther)-diols, les polythioéther-diols et les polycarbonate-diols.

Les polyéther-diols préférés sont, par exemple, les produits de condensation d'oxyde d'éthylène, d'oxyde de propylène ou de tétrahydrofurane, leurs produits de copolymérisation ou de condensation, greffés ou blocs, tels que les mélanges de condensats d'oxyde d'éthylène et de propylène, et les produits de polymérisation d'oléfines, sous haute pression, avec les condensats d'oxyde d'alkylène. Des polyéthers appropriés sont par exemple préparés par condensation d'oxydes d'alkylène et d'alcools polyhydriques, tels que l'éthylèneglycol, le 1,2-propylèneglycol et le 1,4-butanediol.

Les polyester-diols, polyester-amides, polyamide-diols sont de préférence saturés et sont obtenus, par exemple, à partir de la réaction d'acides polycarboxyliques saturés ou insaturés avec des alcools polyhydriques, des diamines ou des polyamines. Pour préparer ces composés, on peut utiliser, par exemple, l'acide adipique, l'acide succinique, l'acide phtalique, l'acide téréphtalique et l'acide maléique. Des alcools polyhydriques appropriés pour préparer les polyesters incluent par exemple l'éthylèneglycol, le 1,2-propylèneglycol, le 1,4-butanediol, le néopentylglycol et l'hexanediol. On peut aussi utiliser des aminoalcools, par exemple l'éthanolamine. Des diamines appropriées pour préparer les amide-polyesters sont l'éthylène-diamine et l'hexaméthylène-diamine.

Des polyacétals appropriés peuvent être préparés, par exemple, à partir de 1,4-butanediol ou d'hexanediol et de formaldéhyde. Des polythioéthers appropriés peuvent être préparés par exemple par réaction de condensation entre des thioglycols seuls ou en combinaison avec d'autres glycols tels que l'éthylèneglycol, le 1,2-propylèneglycol ou avec d'autres composés polyhydroxylés. Les composés polyhydroxylés contenant déjà des groupements uréthannes, des polyols naturels, qui peuvent être davantage modifiés, par exemple, l'huile de castor et les carbohydrates peuvent également être utilisés.

Plus préférentiellement, le composé du groupe (1) est un polyestérol, notamment un polyester-diol formé par la réaction d'au moins un (di)-polyol (1ₐ) et d'au moins un acide (1_{b}). Le (di)- polyol (1ₐ) est en particulier choisi dans le groupe comprenant le néopentylglycol, le 1,4-butanediol, l'hexanediol, l'éthylèneglycol, le diéthylèneglycol, le propylèneglycol, le butylèneglycol, le néopentylglycol et (di)-polyéthylèneglycol. L'acide (1_{b}) est en particulier choisi dans le groupe comprenant l'acide phtalique, l'acide isophtalique, l'acide adipique et l'acide (poly)-lactique.

En tant que composé (2), on peut notamment utiliser un acide hydroxycarboxylique tel que l'acide diméthylol-propanoïque (DMPA) ou un acide 2,2-hydroxyméthyl-carboxylique. En général, le composé (2) est utile en tant que bloc de couplage. En tant que composés (2), on préfère ceux comprenant au moins un poly (acide-(alpha-hydroxycarboxyliquediol)).

Les composés (2) particulièrement préférés sont ceux choisis dans le groupe comprenant l'acide 2,2-di-(hydroxyméthyl)acétique, l'acide 2,2-dihydroxyméthylpropionique, l'acide 2,2-dihydroxyméthylbutyrique, l'acide 2,2-dihydroxyméthylpentanoïque.

Le di- ou polyisocyanate (3) peut être choisi en particulier dans le groupe comprenant l'hexaméthylène-diisocyanate, l'isophorone-diisocyanate (IDPI), le toluylène-diisocyanate, le diphénylméthane-4,4'-diisocyanate (DPMD) et le dicyclohexyl-méthane-4,4'-diisocyanate (DCMD), le méthylène-di-p-phényl-diisocyanate, le méthylène-bis(4-cyclohexylisocyanate), les toluène-diisocyanates, le 1,5-naphtalène-diisocyanate, le 4,4'-diphényl-méthane-diisocyanate, le 2,2'-diméthyl-4,4'-diphénylméthane-diisocyanate, le 1,3-phénylène-diisocyanate, le 1,4-phénylène diisocyanate, des mélanges de 2,4- et de 2,6-toluène-diisocyanates, le 2,2'-dichloro-4,4'-diisocyanato-diphénylméthane, le 2,4-dibromo-1,5-diisocyanato naphtalène, le butane-1,4-diisocyanate, l'hexane-1,6-diisocyanate, le cyclohexane-1,4-diisocyanate.

Le polyuréthane fixant peut être formé à l'aide d'un composé supplémentaire (4) servant en général à allonger sa chaîne. Ces composés (4) peuvent être choisis dans le groupe comprenant notamment les glycols saturés ou insaturés tels que l'éthylèneglycol, le diéthylèneglycol, le néopentylglycol, le triéthylène glycol ; les aminoalcools tels que l'éthanolamine, la propanolamine, la butanolamine ; les amines primaires hétérocycliques, aromatiques, cycloaliphatiques, et aliphatiques ; les diamines ; les acides carboxyliques tels que les acides carboxyliques aliphatiques, aromatiques et hétérocycliques comme les acides oxalique, succinique, glutarique, adipique, sébacique et téréphtalique ; les acides aminocarboxyliques. Les composés (4) préférés sont les diols aliphatiques.

Les polyuréthanes fixants utilisés selon l'invention peuvent également être formés à partir de composés supplémentaires (5) ayant un squelette siliconé tels que les polysiloxanes, les polyalkylsiloxanes ou les polyarylsiloxanes, notamment les polyéthylsiloxanes, les polyméthylsiloxanes et les polyphénylsiloxanes, comportant éventuellement des chaînes hydrocarbonées greffées sur les atomes de silicium.

Les polyuréthanes fixants utilisés avantageusement comprennent un motif répétitif de base répondant à la formule générale (II) :

- O - B - O - CO - NH - R₄ - NH - CO - (II)

dans laquelle :
- B est un groupe hydrocarboné divalent en C₁ à C₃₀, ce groupe étant substitué ou non par un groupement comportant une ou plusieurs fonctions acides carboxyliques et/ou une ou plusieurs fonctions acides sulfoniques, lesdites fonctions acides carboxyliques et/ou sulfoniques étant sous forme libre ou bien neutralisées partiellement ou totalement par une base minérale ou organique, et
- R₄ est un groupe divalent choisi parmi les groupes alkylènes de type aromatique, aliphatique en C₁ à C₂₀, cycloaliphatique en C₁ à C₂₀, ces groupes étant substitués ou non.

Le groupe R₄ est avantageusement choisi parmi les groupes répondant aux formules suivantes :

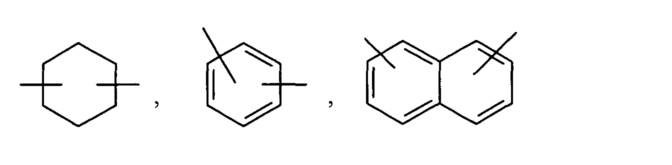

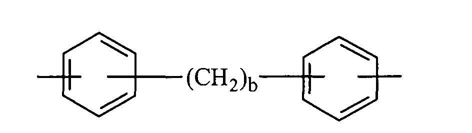

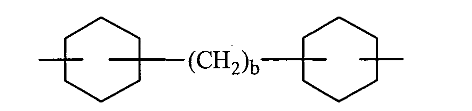

―(CH₂)_{c}―

dans lesquelles b est un nombre entier compris entre 0 et 3, et c un nombre entier compris entre 1 et 20, de préférence compris entre 2 et 12.

En particulier, le groupe R₄ est choisi parmi les groupes hexaméthylène, 4,4'-biphénylèneméthane, 2,4- et/ou 2,6-tolylène, 1,5-naphtylène, p-phénylène, méthylène- 4,4-bis-cyclohexyle et le groupe divalent dérivé de l'isophorone.

Le polyuréthane fixant non associatif utilisé dans la présente invention peut avantageusement comprendre en outre au moins une séquence polysiloxane dont le motif répétitif de base répond par exemple à la formule générale (III) :

- O - P - O - CO - NH - R₅ - NH - CO - (III)

dans laquelle :
- P est un segment polysiloxanique, et
- R₅ est un groupe divalent choisi parmi les groupes alkylènes de type aromatique, aliphatique en C₁ à C₂₀, cycloaliphatique en C₁ à C₂₀, ces groupes étant substitués ou non.

Avantageusement, le segment polysiloxanique P répond à la formule générale (IV) ci-après : 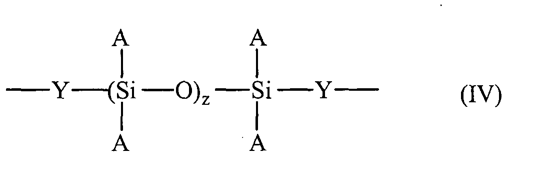 dans laquelle:
- les groupes A, qui peuvent être identiques ou différents, sont choisis parmi d'une part les groupes hydrocarbonés monovalents en C₁ à C₂₀ exempts ou substantiellement exempts d'insaturation éthylénique et, d'autre part, les groupes aromatiques,
- Y représente un groupe hydrocarboné divalent, et
- z représente un nombre entier, choisi de telle sorte que la masse moléculaire moyenne du segment polysiloxane soit comprise entre 300 et 10 000.

En général, le groupe divalent Y est choisi parmi les groupes alkylènes de formule -(CH₂)ₐ- , dans laquelle a représente un nombre entier pouvant être compris entre 1 et 10.

Les groupes A peuvent être choisis parmi les groupes alkyles, en particulier les groupes méthyle, éthyle, propyle, isopropyle, butyle, pentyle, hexyle, octyle, décyle, dodécyle et octadécyle ; les groupes cycloalkyle, en particulier le groupe cyclohexyle ; les groupes aryle, notamment phényle et naphtyle ; les groupes arylalkyle, notamment benzyle et phényléthyle, ainsi que les groupes tolyle et xylyle.

A titre d'exemples de polyuréthane anionique fixant, on peut notamment citer le copolymère acide diméthylolpropionique/ isophorone-diisocyanate/néopentylglycol/polyesterdiols (connu aussi sous le nom de polyuréthane-1, appellation INCI) vendu sous la marque Luviset® PUR par la société BASF, et le copolymère acide diméthylolpropionique/isophorone-diisocyanate/néopentylglycol/polyesterdiols/diamine siliconée (connu aussi sous le nom de polyuréthane-6, appellation INCI) vendu sous la marque Luviset® Si PUR par la société BASF.

Selon l'invention, les polymères fixants anioniques sont de préférence choisis parmi les polyuréthanes anioniques, les copolymères d'acide acrylique tels que les terpolymères acide acrylique/acrylate d'éthyle/N-tertiobutylacrylamide vendus notamment sous la dénomination ULTRAHOLD® STRONG par la société BASF, les copolymères d'acide crotonique tels que les terpolymères acétate de vinyle/tertio-butylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ® par la société ISP, les copolymères d'acide méthacrylique et de méthacrylate de méthyle vendus sous la dénomination EUDRAGIT® L par la société ROHM PHARMA, les copolymères d'acide méthacrylique et d'acrylate d'éthyle vendus sous la dénomination LUVIMER® MAEX ou MAE par la société BASF et les copolymères acétate de vinyle/acide crotonique vendus notamment sous la dénomination LUVISET® CA 66 par la société BASF et les copolymères acétate de vinyle/acide crotonique greffés par du polyéthylèneglycol vendus sous la dénomination ARISTOFLEX® A par la société BASF.

Parmi les polymères fixants anioniques cités ci-dessus, on préfère plus particulièrement utiliser dans le cadre de la présente invention le polyuréthane siliconé vendu sous la dénomination LUVISET® Si PUR, les copolymères méthylvinyléther/anhydride maléique monoestérifiés vendus sous la dénomination GANTREZ® ES 425 par la société ISP, les terpolymères acide acrylique/acrylate d'éthyle/N-tertiobutylacrylamide vendus sous la dénomination ULTRAHOLD® STRONG par la société BASF, les copolymères d'acide méthacrylique et de méthacrylate de méthyle vendus sous la dénomination EUDRAGIT® L par la société ROHM PHARMA, les terpolymères acétate de vinyle/tertio-butylbenzoate de vinyle/acide crotonique et les terpolymères acide crotonique/acétate de vinyle/néododécanoate de vinyle vendus sous la dénomination Résine 28-29-30 par la société NATIONAL STARCH, les copolymères d'acide méthacrylique et d'acrylate d'éthyle vendus sous la dénomination LUVIMER® MAEX OU MAE par la société BASF, les terpolymères vinylpyrrolidone/acide acrylique/méthacrylate de lauryle vendus sous la dénomination ACRYLIDONE® LM par la société ISP.

Le ou les polymères fixants anioniques, tels que définis ci-dessus sont utilisés selon l'invention en une proportion allant de 0,05 à 25 % de préférence allant de 0,1 à 20 % en poids, mieux encore de 0,5 à 10 % en poids par rapport au poids total de la composition.

Les silicones oxyalkylénées en positions α et ω de la chaîne siliconée utilisables dans la composition selon l'invention sont des polymères organosiliciés à squelette linéaire, substitués aux deux extrémités de la chaîne principale par des groupements oxyalkylène reliés aux atomes de Si par l'intermédiaire d'un groupement hydrocarboné. De préférence, la chaîne principale ne comprend pas de groupement oxyalkylène pendant.

De préférence, la silicone oxyalkylénée en positions α et ω de la chaîne siliconée répond à la formule (V) suivante : 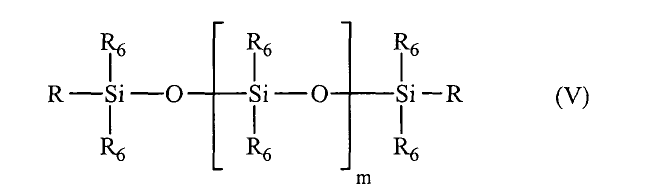 dans laquelle :
- R = -(CH₂)ₚ-O-(C₂H₄O)ₓ(C₃H₆O)_{y}R₇
   où :
- R₇ représente H, CH₃ ou CH₂CH₃,
- p est un entier allant de 1 à 5, x varie de 1 à 100, y varie de 0 à 50,
- les motifs (C₂H₄O) et (C₃H₆O) peuvent être répartis de façon aléatoire ou par blocs,
- les symboles R₆ représentent chacun, indépendamment l'un de l'autre, un groupe alkyle en C₁-C₃ ou un groupe phényle,
- 5 ≤ m ≤ 300.

De préférence, la silicone oxyalkylénée en positions α et ω de la chaîne siliconée, utilisée selon la présente invention répond à la formule générale (V) pour laquelle tous les symboles R₆ représentent des groupes méthyle et :
- p va de 2 à 4,
- x va de 3 à 100,
- m va de 50 à 200.

De préférence encore, le poids moléculaire moyen de R va de 800 à 2600.

De préférence, le rapport en poids des motifs C₂H₄O par rapport aux motifs C₃H₆O va de 100 :10 à 20 :80, mieux encore il est d'environ 42/58.

De préférence encore, R₇ représente un groupe méthyle.

De façon plus préférentielle encore, la composition selon l'invention comprend la silicone oxyalkylénée en positions α et ω de la chaîne siliconée, de formule suivante : 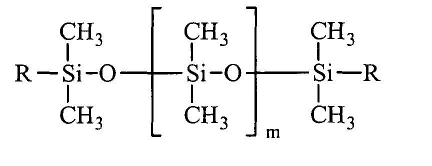 dans laquelle :
- m = 100,
- R = (CH₂)₃-O-(C₂H₄O)ₓ(C₃H₆O)_{y}-CH₃, où x va de 3 à 100, y va de 1 à 50, le rapport en poids du nombre de C₂H₄O sur le nombre de C₃H₆O étant d'environ 42/58, le poids moléculaire moyen de R allant de 800 à 1500.

Parmi les produits du commerce pouvant contenir tout ou partie des silicones oxyalkylénées en positions α et ω de la chaîne siliconée, utilisables selon l'invention, on peut notamment citer ceux vendus sous les dénominations de « Abil EM 97 » par la Société Goldschmidt, ou encore de « KF 6009 », « X22-4350 », « X22-4349 » ou « KF 6008 » par la Société Shin Etsu.

La ou les silicones oxyalkylénées en positions α et ω de la chaîne siliconée, telles que définies ci-dessus sont utilisées selon l'invention en une proportion allant de 0,01 à 20 % de préférence allant de 0,05 à 10 % en poids, mieux encore de 0,1 à 5 % en poids par rapport au poids total de la composition.

A titre d'agent propulseur utilisable dans la présente invention, on peut notamment citer le diméthyléther, les alcanes en C₃₋₅, le 1,1-difluoroéthane, les mélanges de diméthyléther et d'alcanes en C₃₋₅, les mélanges de 1,1-difluoroéthane et de diméthyléther et/ou d'alcanes en C₃₋₅, et plus particulièrement le diméthyléther seul ou en mélange.

L'agent propulseur est notamment présent en une quantité allant de 25 à 90 % en poids, de préférence de 35 à 80 % en poids par rapport au poids total de la composition.

Le milieu cosmétiquement acceptable comprend de l'eau et/ou un ou plusieurs solvants cosmétiquement acceptable notamment choisi parmi les alcools inférieurs en C₁-C₄, comme l'éthanol, l'isopropanol, le tertio-butanol et le n-butanol ; les polyols comme le propylèneglycol ; les éthers de polyols ; l'acétone ; et leurs mélanges. Le solvant particulièrement préféré dans l'invention est l'éthanol.

Ce milieu cosmétiquement acceptable comprend de préférence une quantité d'eau inférieure à 20 % en poids de la composition.

La composition selon l'invention peut comprendre en outre au moins un additif cosmétique usuel choisi parmi les silicones différentes de celles définies ci-dessus, sous forme soluble ou dispersée, les polymères fixants non ioniques, amphotères ou cationiques, les agents épaississants, les nacrants, les opacifiants, les filtres UV, les sucres, les parfums, les huiles minérales, végétales et/ou synthétiques, les esters d'acides gras, les colorants, les particules minérales ou organiques, naturelles ou synthétiques, les conservateurs et les agents de stabilisation du pH.

L'homme de métier veillera à choisir les éventuels additifs et leur quantité de manière à ce qu'ils ne nuisent pas aux propriétés des compositions de la présente invention.

Ces additifs sont notamment présents dans la composition selon l'invention en une quantité allant de 0 à 50 % en poids par rapport au poids total de la composition.

Les compositions conformes à l'invention sont conditionnées dans un dispositif aérosol usuel en cosmétique. Les compositions pulvérisées se présentent sous la forme d'un spray.

Les compositions conformes à l'invention, pulvérisées à partir du dispositif aérosol, peuvent être utilisées en application rincée ou non, comme compositions de fixation et/ou de maintien des cheveux, compositions de soin de cheveux, shampooings, compositions de conditionnement des cheveux, telles que des compositions destinées à apporter de la douceur aux cheveux, ou encore des compositions de maquillage des cheveux.

Selon un mode de réalisation préféré de l'invention, la composition pulvérisée à partir du dispositif aérosol peut être utilisée comme produit de coiffage non rincé, en particulier pour la mise en forme des cheveux.

La présente invention concerne également un procédé de traitement cosmétique des cheveux qui consiste à appliquer une quantité efficace d'une composition telle que décrite ci-dessus, sur les cheveux et à rincer ou non après un éventuel temps de pose.

Une fois appliquée sur les cheveux, la composition selon l'invention conduit à une mise en forme qui peut, par ailleurs, s'éliminer facilement tout en laissant les cheveux aisément démêlable.

L'invention concerne aussi l'utilisation du procédé pour améliorer le démêlage après élimination de la composition.

L'exemple suivant illustre la présente invention.

### Exemple

On a préparé un produit de coiffage à partir des ingrédients suivants, les pourcentages étant exprimés en poids :

| | |
|---|---|
| Silicone Abil EM 97 | 0,1 % |
| Copolymère acétate de vinyle/ tertiobutylbenzoate de vinyle/acide crotonique | 4,9% |
| 2-Amino-2-méthyl-1-propanol | 0,2 % |
| Eau | 16,4 % |
| Alcool éthylique | 33,4 % |
| Diméthyléther | 45 % |

Cette composition est conditionnée dans un dispositif aérosol.

Pulvérisée sur cheveux secs après mise en forme de la coiffure, cette composition permet de maintenir la forme sans durcissement excessif et s'élimine facilement au brossage en laissant des cheveux aisément démêlables, souples et lisses.

Des résultats analogues peuvent être obtenus en remplaçant le propulseur par le 1,1-difluoroéthane et le niveau de fixation peut être ajusté en modifiant la concentration du polymère anionique et/ou en lui ajoutant un second polymère anionique tel que le Luviset Si Pur de BASF.

## Revendications

1. Composition conditionnée dans un dispositif aérosol, comprenant dans un milieu cosmétiquement acceptable, au moins un polymère fixant anionique, au moins une silicone oxyalkylénée en positions α et ω de la chaîne siliconée, et au moins un agent propulseur.

2. Composition selon la revendication 1, dans laquelle le polymère fixant anionique est choisi parmi les homopolymères ou copolymères d'acide acrylique et méthacrylique ou leurs sels, les copolymères d'acide crotonique, les copolymères d'acides ou d'anhydrides carboxyliques monoinsaturés en C₄-C₈, les polyacrylamides à groupements carboxylates, les homopolymères ou copolymères à groupes sulfoniques, et les polyuréthanes anioniques.

3. Composition selon la revendication 2, dans laquelle le polymère fixant anionique est choisi parmi les polyuréthanes anioniques, les copolymères d'acide acrylique, les copolymères d'acide crotonique, les polymères d'acides ou d'anhydrides maléique, fumarique, itaconique avec des esters vinyliques, des éthers vinyliques, des halogénures vinyliques, des dérivés phénylvinyliques, l'acide acrylique et ses esters, les copolymères d'acide méthacrylique et de méthacrylate de méthyle, les copolymères d'acide méthacrylique et d'acrylate d'éthyle, les copolymères acétate de vinyle/acide crotonique et les copolymères acétate de vinyle/acide crotonique greffés par du polyéthylèneglycol.

4. Composition selon la revendication 3, dans laquelle le polymère fixant anionique est choisi parmi le copolymère acide diméthylolpropionique/isophorone-diisocyanate/néopentylglycol/polyesterdiols/diamine siliconée, les copolymères méthylvinyléther/anhydride maléique monoestérifiés, les terpolymères acide acrylique/acrylate d'éthyle/N-tertiobutylacrylamide, les copolymères d'acide méthacrylique et de méthacrylate de méthyle, les terpolymères acétate de vinyle/tertio-butylbenzoate de vinyle/acide crotonique, les terpolymères acide crotonique/acétate de vinyle/néododécanoate de vinyle, les copolymères d'acide méthacrylique et d'acrylate d'éthyle, et les terpolymères vinylpyrrolidone/acide acrylique/méthacrylate de lauryle.

5. Composition selon l'une quelconque des revendications précédentes, dans laquelle le polymère fixant anionique est utilisé en une proportion allant de 0,05 à 25 % de préférence allant de 0,1 à 20 % en poids par rapport au poids total de la composition.

6. Composition selon l'une quelconque des revendications précédentes, dans laquelle la silicone oxyalkylénée en positions α et ω répond à la formule (V) suivante : 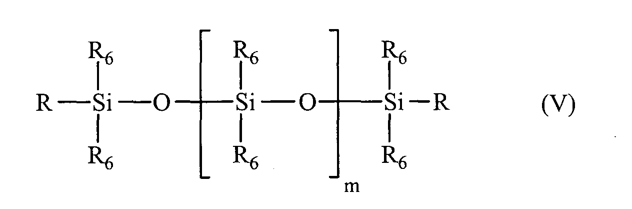 dans laquelle :
R = -(CH₂)ₚ-O-(C₂H₄O)ₓ(C₃H₆O)_{y} R₇
où :
- R₇ représente H, CH₃ ou CH₂CH₃,
- p est un entier allant de 1 à 5, x varie de 1 à 100, y varie de 0 à 50,
- les motifs (C₂H₄O) et (C₃H₆O) peuvent être répartis de façon aléatoire ou par blocs,
- les symboles R₆ représentent chacun, indépendamment l'un de l'autre, un groupe alkyle en C₁-C₃ ou un groupe phényle,
- 5 ≤ m ≤ 300.

7. Composition selon la revendication 6, dans laquelle tous les symboles R₆ représentent des groupes méthyle et :
- p va de 2 à 4,
- x va de 3 à 100,
- m va de 50 à 200.

8. Composition selon la revendication 6 ou 7, dans laquelle le rapport en poids des motifs C₂H₄O par rapport aux motifs C₃H₆O va de 100 : 10 à 20 : 80.

9. Composition selon l'une quelconque des revendications 6 à 8, dans laquelle la silicone oxyalkylénée en positions α et ω de la chaîne siliconée répond à la formule suivante : 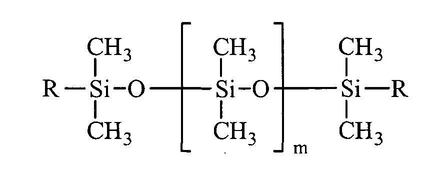 dans laquelle :
- m = 100,
- R = (CH₂)₃-O-(C₂H₄O)ₓ(C₃H₆O)_{y}-CH₃,
- x va de 3 à 100, et y va de 1 à 50,
- le rapport en poids du nombre de C₂H₄O sur le nombre de C₃H₆O étant d'environ 42/58, et
- le poids moléculaire moyen de R allant de 800 à 1500.

10. Composition selon l'une quelconque des revendications précédentes, dans laquelle la silicone oxyalkylénée en positions α et ω de la chaîne siliconée est utilisée en une proportion allant de 0,01 à 20 % de préférence allant de 0,05 à 10 % en poids par rapport au poids total de la composition.

11. Composition selon l'une quelconque des revendications précédentes, dans laquelle l'agent propulseur est choisi parmi le diméthyléther, les alcanes en C₃₋₅, le 1,1-difluoroéthane, les mélanges de diméthyléther et d'alcanes en C₃₋₅, les mélanges de 1,1-difluoroéthane et de diméthyléther et/ou d'alcanes en C₃₋₅.

12. Composition selon la revendication 11, dans laquelle l'agent propulseur est le diméthyléther seul ou en mélange, ou le 1,1-difluoroéthane.

13. Composition selon l'une quelconque des revendications précédentes, dans laquelle l'agent propulseur est contenu en une quantité allant de 25 à 90 % en poids, de préférence de 35 à 80 % en poids par rapport au poids total de la composition.

14. Composition selon l'une quelconque des revendications précédentes, dans laquelle le milieu cosmétiquement acceptable comprend de l'eau et/ou au moins un solvant cosmétiquement acceptable.

15. Composition selon la revendication 14, **caractérisée en ce que** le solvant est choisi parmi les alcools inférieurs en C₁₋₄, les polyols, les éthers de polyols, l'acétone et leurs mélanges.

16. Composition selon la revendication 14, **caractérisée en ce que** l'eau représente moins de 20 % en poids de la composition.

17. Composition selon l'une quelconque des revendications précédentes, **caractérisée en ce qu'**elle comprend en outre au moins un additif cosmétique choisi parmi les silicones différentes de celles définies aux revendications 1, 6 à 9, sous forme soluble ou dispersée, les polymères fixants non ioniques, amphotères ou cationiques, les agents épaississants, les nacrants, les opacifiants, les filtres UV, les sucres, les parfums, les huiles minérales, végétales et/ou synthétiques, les esters d'acides gras, les colorants, les particules minérales ou organiques, naturelles ou synthétiques, les conservateurs et les agents de stabilisation du pH.

18. Utilisation de la composition selon l'une quelconque des revendications 1 à 17, pulvérisée à partir du dispositif aérosol, comme produit de coiffage, de préférence non rincé.

19. Utilisation selon la revendication 18, pour la mise en forme des cheveux.

20. Procédé de traitement cosmétique des cheveux, **caractérisé en ce que** l'on applique la composition selon l'une quelconque des revendications 1 à 17, sur les cheveux et que l'on rince ou non après un éventuel temps de pose.

21. Utilisation du procédé selon la revendication 20, pour améliorer le démêlage après élimination de la composition.
